# EUROPEAN PATENT APPLICATION

(11) **EP 4 501 405 A1**
(43) Date of publication of application: **05.02.2025**
(21) Application number: 23781023.9
(22) Date of filing: 30.03.2023
(51) Int. Cl.: A61P 17/00, A61Q 19/00, A61Q 19/08, A23L 33/13, A61K 8/60, A61K 31/706

(54) **COLLAGEN PRODUCTION PROMOTER, COSMETIC COMPOSITION OR EXTERNAL PREPARATION FOR SKIN, AND PREPARATION FOR ORAL ADMINISTRATION**

(30) Priority: 30.03.2022 JP 2022056619
(71) Applicant: YAMASA CORPORATION, Choshi-shi, Chiba 288-0056 (JP)
(72) Inventor: ISHIGE,Kazuya, Choshi-shi, Chiba 288-0056 (JP)
(74) Representative: Maiwald GmbH
(86) International application number: PCT/JP2023/013438
(87) International publication number: WO 2023/191015

(57) **Abstract**

A collagen production promoter contains at least one pyrimidine nucleotide or precursor thereof as an active ingredient.

## Description

### [Technical Field]

The present invention relates to a collagen production promoter, a cosmetic composition or an external preparation for skin, and a preparation for oral administration, which promote collagen production.

### [Background Art]

In today's aging society, healthy longevity as well as improvement of QOL (Quality of Life) have become issues. Skin beauty in the anti-aging field is very important for improving the quality of life of elderly people, and so there is growing interest.

With aging, the skin undergoes appearance changes such as spots, wrinkles, and flabbiness. These appearance changes significantly reduce the quality of life of the elderly people. Collagen loss with aging has been noted as one of the major factors contributing to these appearance changes.

The skin consists of three layers: epidermis, dermis, and subcutaneous tissue, with the majority of the dermis composed of collagen. Collagen molecules have a triple helical structure having three intertwined polypeptide chains with a molecular weight of approximately 100,000. It is believed that the collagen molecules provide elasticity, water absorption, and swelling properties to dermal cells (Non-Patent Literature 1).

Collagen is known to decrease with aging. Known causes are glycation, which is the binding of collagen to sugar, and the production of collagen-degrading enzymes triggered by ultraviolet light stimulation of epidermal cells, and the like. As collagen decreases, cells lose elasticity, water absorption, and swelling properties. In other words, changes such as loss of skin elasticity, loss of water retention, and skin laxity are generated.

Since a decrease in collagen causes appearance changes such as spots, wrinkles, and flabbiness, studies have been conducted to increase an amount of collagen in the skin in order to prevent those aging phenomena. One example is the oral ingestion of collagen and its use as a cosmetic composition or external preparation for skin. However, it is believed that the oral ingestion of collagen does not directly increase the amount of collagen in the skin, because collagen is decomposed in the digestive tract before being absorbed. As for the use of collagen as the cosmetic composition or the external preparation for skin, it is believed that collagen having a high molecular weight cannot be penetrated into the skin, and therefore has no effect of increasing the amount of collagen in the skin.

Therefore, in recent years, attention has focused on the collagen-producing ability of fibroblasts, and methods to increase the amount of collagen in the skin by stimulating collagen production by fibroblasts have been attracting attention.

Patent Literature 1 describes a collagen production promoting composition containing a purine-based nucleic acid-related substance and a pyrimidine-based nucleic acid-related substance. However, the invention substantially disclosed in Patent Literature 1 is the collagen production promoting composition using the combination of adenylic acid and uridylic acid, in which the main active substance is adenylic acid. There is no substantial disclosure regarding the use of uridylic acid alone and the use of cytidylic acid in Patent Literature 1. Furthermore, Patent Literature 1 discloses that uridylic acid alone has no effect of promoting collagen production.

In addition, Examples shown in Patent Literature conduct a test by directly applying a sample to cultured human skin fibroblasts. However, fibroblasts are present in the dermis, so that the external composition cannot directly be delivered to the fibroblasts. In other words, the experimental system described in Patent Literature 1 does not precisely evaluate the phenomena that occur in the skin when the sample is used as an external preparation for skin.

### [Citation List]

### [Patent Literature]

[PTL 1]
WO 2005/034902 A1

### [Non-Patent Literature]

[NON-PTL1]
Chemistry and Education, Vol. 40, No. 10 (1992), P. 673

### [Summary of Invention]

### [Technical Problem]

An object of the present invention is to provide a novel and highly safe collagen production promoter, a cosmetic composition or external preparation for skin, and a preparation for oral administration, which have an effect of promoting collagen production.

### [Solution to Problem]

As a result of intensive studies to achieve the above object, surprisingly, the present inventors have firstly found that pyrimidine nucleotides or precursors thereof, which were previously thought to have no effect of promoting collagen production, have an attribute of promoting collagen production in fibroblasts by themselves through epidermal cells, and they have completed the present invention.

Thus, the present invention is a collagen production promoter comprising at least one pyrimidine nucleotide or precursor thereof as an active ingredient.

The present invention is also a cosmetic composition or an external preparation for skin, for promoting collagen production, wherein the cosmetic composition or the external preparation for skin comprises at least one pyrimidine nucleotide or precursor thereof as an active ingredient.

Furthermore, the present invention is a preparation for oral administration for promoting collagen production, wherein the preparation comprises at least one pyrimidine nucleotide or precursor thereof as an active ingredient.

### [Advantageous Effects of Invention]

The collagen production promoter, the cosmetic composition or external preparation for skin, and the preparation for oral administration according to the present invention have an effect of promoting collagen production, and are novel and highly safe. In particular, the cosmetic composition or external preparation for skin, and the preparation for oral administration according to the present invention can promote collagen production in fibroblasts through epidermal cells.

### [Brief Description of Drawings]

[FIG. 1]

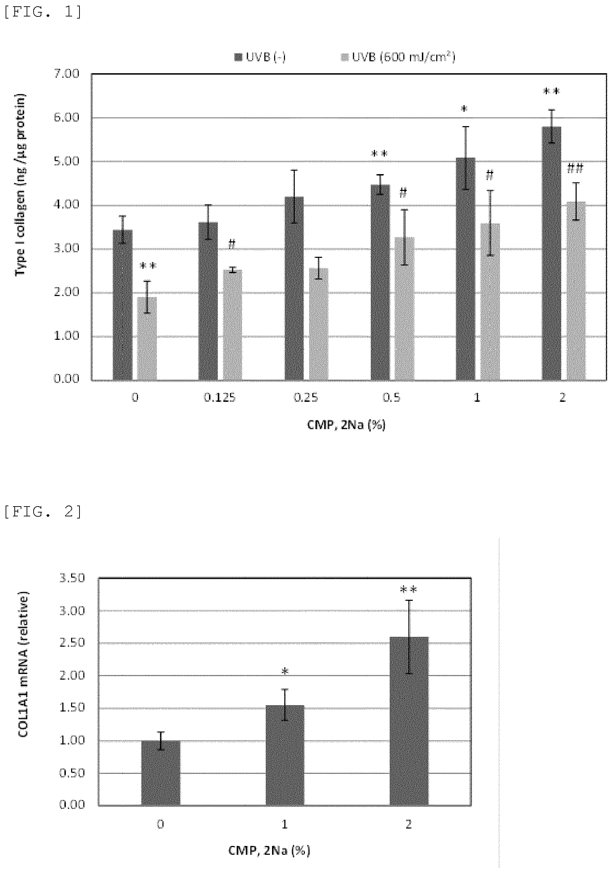

   FIG. 1 shows quantified results of an amount of type I collagen (ng/µg protein) quantified in Example 1. In the figure, the UVB (-) shown in the dark gray color means measured results of samples that were not subjected to a UV treatment. The UVB (600 mJ/cm²) shown in the grayish white color means measured results of samples that were subjected to a UVB treatment by the method described in Example 1. Each value on the horizontal axis means a CMP, 2Na concentration (w/v%) in each sample. The symbol * means that the p value is smaller than 0.05 when compared with the result of the UVB (-) CMP, 2Na at a concentration of 0 (w/v%) in Student's t-test, and the symbol ** means that the p value is smaller than 0.01 when compared with the result of the UVB (-) CMP, 2Na at a concentration of 0 (w/v%) in Student's t-test. The symbol # means that the p value is smaller than 0.05 when compared with the result of the UVB (600 mJ/cm²) CMP, 2Na at a concentration of 0 (w/v%) in Student's t-test, and the symbol ## means that the p value is smaller than 0.01 when compared with the result of the UVB (600 mJ/cm²) CMP, 2Na at a concentration of 0 (w/v%) in Student's t-test.
[FIG. 2]
   FIG. 2 shows results of analysis for mRNA expression of COL1A1 quantified in Example 2. Each value on the horizontal axis means a concentration (w/v%) of CMP, 2Na in each sample. Each value on the vertical axis means a relative expression level when the expression level of COL1A1 at a CMP, 2Na concentration of 0 (w/v%) is 1.00. The symbol * means that the p value is smaller than 0.05 when compared with the result at a CMP, 2Na concentration of 0 (w/v%) in Student's t-test, and the symbol ** means that the p value is smaller than 0.01 when compared with the result at a CMP, 2Na concentration of 0 (w/v%) in Student's t-test.
[FIG. 3]

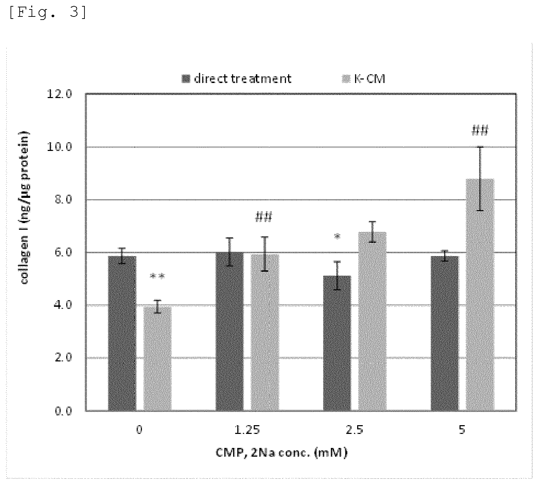

   FIG. 3 shows quantified results of an amount of type I collagen (ng/µg protein) quantified in Example 3 after a CMP, 2Na treatment. In the figure, the "direct treatment" shown in the dark gray color means measured results of samples in which the samples were directly treated with normal human fibroblasts. The "K-CM" shown in the grayish white color means measured results of samples in which a culture supernatant of normal human epidermal cells was treated with normal human fibroblasts. Each value on the horizontal axis means the CMP, 2Na concentration (mM) in each sample. The symbol * means that the p value is smaller than 0.05 when compared with the result of the direct treatment at a CMP, 2Na concentration of 0 (mM) in Student's t-test, and the symbol ** means that the p value is smaller than 0.01 when compared with the result of the direct treatment at a CMP, 2Na concentration of 0 (mM) in Student's t-test. The symbol # means that the p value is smaller than 0.05 when compared with the result of K-CM at a CMP, 2Na concentration of 0 (mM) in Student's t-test, and the symbol ## means that the p value is smaller than 0.01 when compared with the result of K-CM at a CMP, 2Na concentration of 0 (mM) in Student's t-test.
[FIG. 4]

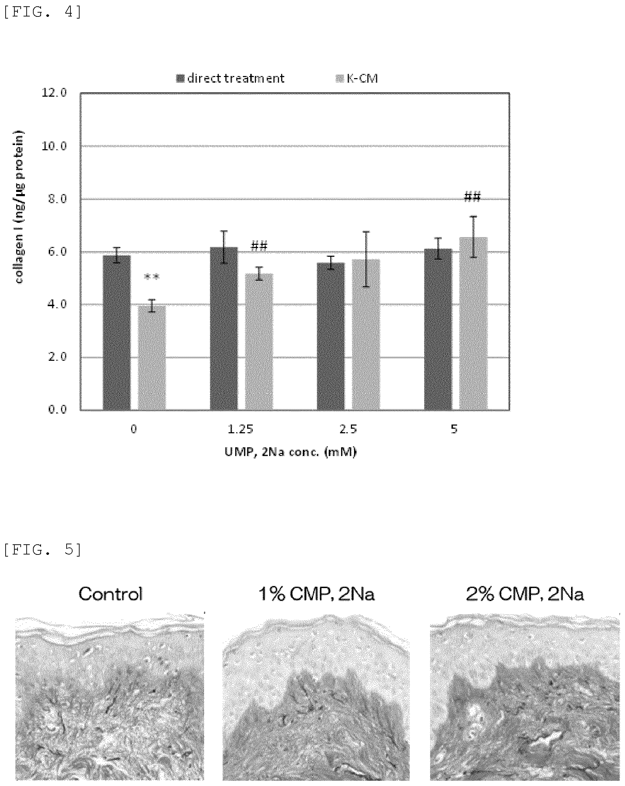

   FIG. 4 shows quantified results of an amount of type I collagen (ng/µg protein) quantified in Example 3 when treated with UMP, 2Na. In the figure, the "direct treatment" shown in the dark gray color means measured results of samples in which the samples were directly treated with normal human fibroblasts. The "K-CM" shown in the grayish white color means measured results of samples in which normal human fibroblasts were treated with the culture supernatant of normal human epidermal cells. Each value on the horizontal axis means the concentration (mM) of UMP, 2Na in each sample. The symbol * means that the p value is smaller than 0.05 when compared with the result of the direct treatment at a UMP, 2Na concentration of 0 (mM) in Student's t-test, and the symbol ** means that the p value is smaller than 0.01 when compared with the result of the direct treatment at a UMP, 2Na concentration of 0 (mM) in Student's t-test. The symbol # means that the p value is smaller than 0.05 when compared with the results of K-CM at a UMP, 2Na concentration of 0 (mM) in Student's t-test, and the symbol ## means that the p value is smaller than 0.01 when compared with the results of K-CM at a UMP, 2Na concentration of 0 (mM) in Student's t-test.
[FIG. 5]
   FIG. 5 shows photographs of results of elastica-van Gieson staining described in Example 4. The dark grey areas in the dermis in the photographs represent collagen fibers. In the figure, the "Control" shows a result of a sample treated with CMP, 2Na at a concentration of 0 (w/v%). The "1% CMP, 2Na" shows a result of a sample treated with CMP, 2Na at a concentration of 1.0 (w/v%), and the "2% CMP, 2Na" shows a result of a sample treated with CMP, 2Na at a concentration of 2.0 (w/v%).

### [Description of Embodiments]

The present invention relates to a collagen production promoter, a cosmetic composition or external preparation for skin, and a preparation for oral administration, which are for promoting collagen production and contain at least one pyrimidine nucleotide or precursor thereof as an active ingredient.

As used herein, the "collagen production promoter" is a concept including a form of a composition that contains at least one pyrimidine nucleotide or precursor thereof as an active ingredient and further contains other ingredients. Similarly, as used herein, the "external preparation for skin" is a concept including a form of a composition that contains at least one pyrimidine nucleotide or precursor thereof as an active ingredient and further contains other ingredients.

As used herein, the pyrimidine nucleotide means cytidylic acid and/or uridylic acid.

The cytidylic acid (cytidine monophosphate, cytidine 5'-phosphate, CMP) is a compound represented by CAS Registry Number 63-37-6. When cytidylic acid is mentioned herein, salts of cytidylic acid are also included.

When a mass of cytidylic acid is described herein, it represents a mass when converted to disodium cytidylate (CMP,2Na). When a concentration (%) of a cytidylic acid solution is mentioned herein, it is a mass volume percent concentration (w/v%) unless otherwise specified, and a mass converted to CMP,2Na is used as the mass of cytidylic acid. If a salt other than the disodium salt is selected, or if it is a free acid that does not form a salt, it is a mass when converted to CMP,2Na, based on the amount of substance of the cytidylic acid.

Uridylic acid (uridine monophosphate, uridine 5'-phosphate, UMP) is a compound represented by CAS registration number 58-97-9. When the term "uridylic acid" is used herein, it is concept also including salts of uridylic acid.

When a mass of uridylic acid is described herein, it represents a mass when converted to disodium uridylate (UMP,2Na). When a concentration (%) of uridylic acid is mentioned herein, it is a mass volume percent concentration (w/v%) unless otherwise specified, and a mass converted to UMP,2Na is used as the mass of uridylic acid. If a salt other than the disodium salt is selected, and if it is a free acid that does not form a salt, it is a mass when converted to UMP,2Na, based on the amount of substance of the uridylic acid.

As used herein, the pyrimidine nucleotide precursor means a compound that can be metabolized to the pyrimidine nucleotide, i.e., cytidylic acid and/or uridylic acid. Whether a compound is included in the pyrimidine nucleotide precursor is determined by the presence or absence of knowledge that the compound is converted to the pyrimidine nucleotide. Specifically, cytidine diphosphate, cytidine triphosphate, uridine diphosphate, and uridine triphosphate, which are known to be degraded to cytidylic acid and/or uridylic acid by the action of ectonucleotidases and like (Isao Matsuoka, "Ectonucleotidase in Nervous System", Japanese journal of Clinical Chemistry 33: 11-18, 2004), and cytidine, cytosine, uridine, and uracil, which are known to be phosphorylated to cytidylic acid and/or uridylic acid by the action of kinases (A Orengo, "Regulation of enzymic activity by metabolites. I. Uridine-cytidine kinase of Novikoff ascites rat tumor", J Biol Chem. 1969 Apr 25; 244(8): 2204-9.) are exemplified as the pyrimidine nucleotide precursors as used herein.

As used herein, the effect of promoting collagen production according to the present invention is evaluated by quantifying an amount of type I collagen. A specific method for the quantification will be described in Examples below.

Examples of the pyrimidine nucleotide or precursor thereof in the present invention include, as described above, cytidine, cytosine, cytidylic acid, cytidine diphosphate, cytidine triphosphate, uridine, uracil, uridylic acid, uridyl diphosphate, and uridyl triphosphate. Among them, cytidylic acid and uridylic acid are preferable.

The concept of cytidylic acid as used herein includes salts as described above. The salts of cytidylic acid include alkali metal salts such as sodium salts and potassium salts; alkaline earth metal salts such as calcium salts, magnesium salts and barium salts; basic amino acid salts such as arginine and lysine; ammonium salts such as ammonium salts and tricyclohexylammonium salts; and various alkanolamine salts such as monoethanolamine salts, diethanolamine salts, triethanolamine salts, monoisopropanolamine salts, diisopropanolamine salts and triisopropanolamine salts. Among them, the alkali metal salts such as sodium salts are preferred. Specific examples of the alkali metal salts include monosodium cytidylate and disodium cytidylate. Disodium cytidylate is preferred in terms of handling.

The concept of uridylic acid as used herein includes salts as described above. The salts of uridylic acid include alkali metal salts such as sodium salts and potassium salts; alkaline earth metal salts such as calcium salts, magnesium salts and barium salts; basic amino acid salts such as arginine and lysine; ammonium salts such as ammonium salts and tricyclohexylammonium salts; and various alkanolamine salts such as monoethanolamine salts, diethanolamine salts, triethanolamine salts, monoisopropanolamine salts, diisopropanolamine salts and triisopropanolamine salts. Among them, the alkali metal salts such as sodium salts are preferred. Specific examples of the alkali metal salts include monosodium uridylate and disodium uridylate. Disodium uridylate is preferred in terms of handling.

The content of the pyrimidine nucleotide or the precursor thereof in the collagen production promoter, the cosmetic composition, the external preparation for skin, and the preparation for oral administration according to the present invention is preferably 0.025 (w/v%) or more. In particular, the content is preferably 0.046 (w/v%) or more in terms of significantly promoting collagen production. Further, since the effect of promoting collagen production is exerted in a concentration-dependent manner, the content is preferably 0.075 (w/v%) or more, and more preferably 0.1 (w/v%) or more, and even more preferably 0.125 (w/v%) or more, and still more preferably 0.25 (w/v%) or more, and even more preferably 0.5 (w/v%) or more, and particularly preferably 1.0 (w/v%) or more. Also, from the viewpoint of ease of handling when it is used as various agents or compositions, the content of pyrimidine nucleotide or the precursor thereof is preferably 10 (w/v%) or less.

In the collagen production promoter, the cosmetic composition or external preparation for skin, and the preparation for oral administration according to the present invention, the pyrimidine nucleotide or the precursor thereof as the active ingredient may be used alone, or two or more types of pyrimidine nucleotides or precursors thereof may be simultaneously used, or the pyrimidine nucleotide or the precursor thereof may be used in combination with other active ingredients.

There is no particular limitation on the origins of the pyrimidine nucleotides or precursors thereof, and those derived from natural products such as yeast, bacteria, fish and shellfish, animals, and plants are suitable.

The collagen production promoter, the cosmetic composition, the external preparation for skin, and the preparation for oral administration according to the present invention may be prepared in various forms by combining pharmaceutically or cosmetically acceptable bases or carriers in addition to the above ingredients. Conventionally known bases and carriers can be used as the pharmaceutically or cosmetically acceptable bases and carriers. Also, in the case of cosmetic composition or the external preparation for skin according to the present invention, it may optionally contain various known ingredients that will be formulated in external compositions applied to the skin or mucous membranes, such as cosmetics, external pharmaceuticals and quasi-drugs. Such ingredients include, for example, surfactants, coloring matters (dyes, pigments), fragrances, preservatives, bactericides (antibacterial agents), thickening agents, antioxidants, sequestering agents, cooling agents, deodorants, moisturizers, UV absorbers, UV scattering agents, vitamins, plant extracts, skin astringents, anti-inflammatory agents (antiphlogistic agents), whitening agents, cell activators, vasodilator agents, blood circulation promoters, skin function enhancers, and the like.

Among the above ingredients, specific examples of the surfactants include anionic surfactants such as higher fatty acid soaps, alkyl sulfate ester salts, polyoxyethylene alkyl ether sulfates, alkyl ether phosphate ester salts, N-acylamino acid salts, and acyl N-methyl taurine salts; cationic surfactants such as alkyltrimethylammonium chloride, and dialkyldimethylammonium chloride; amphoteric surfactants such as alkyl dimethyl amino acetic acid betaine, alkyl amido dimethyl amino acetic acid betaine, and 2-alkyl-N-carboxy-N-hydroxyimidazolinium betaine; nonionic surfactants such as polyoxyethylene type, polyhydric alcohol ester type, and ethylene oxide-propylene oxide block copolymers. Further, surfactants belonging to polymer surfactants or natural surfactants can also be used without limitation.

Specific examples of preservatives include ethyl paraoxybenzoate, salicylic acid, and sorbic acid. Specific examples of the thickening agent include xanthan gum, sodium carboxymethylcellulose, and carboxyvinyl polymers. Specific examples of the sequestering agent include sodium salt of ethylenediaminetetraacetic acid, phosphoric acid, and citric acid.

Specific examples of the moisturizers include polyethylene glycol, propylene glycol, glycerin, 1,3-butylene glycol, xylitol, sorbitol, maltitol, chondroitin sulfate, hyaluronic acid, mucoitinsulfuric acid, charonic acid, atelocollagen, sodium lactate, bile salts, dlpyrrolidone carboxylate, short-chain soluble collagen, diglycerin (EO) PO adducts, chestnut rose extracts, yarrow extracts, and melilot extracts.

Specific examples of the vitamins include classes of vitamin A such as vitamin A oil, retinol, retinol acetate, and retinol palmitate; classes of vitamin B2 such as riboflavin, riboflavin butyrate, and flavin adenine nucleotides; classes of vitamin B6 such as pyridoxine hydrochloride, and pyridoxine dioctanoate; classes of vitamin C such as L-ascorbic acid, L-ascorbyl dipalmitate, L-ascorbic acid-2-sodium sulfate, and dl-α-tocopherol-L-ascorbic acid phosphate diester dipotassium; pantothenic acids such as calcium pantothenate, D-pantothenyl alcohol, pantothenyl ethyl ether, and acetyl pantothenyl ethyl ether: classes of vitamin D such as ergocalciferol and cholecalciferol; nicotinic acids such as nicotinic acid, benzyl nicotinate, and nicotinamide; classes of vitamin E such as dl-α-tocopherol, dl-α-tocopherol acetate, dl-α-tocopherol nicotinate, and dl-α-tocopherol succinate; and vitamin P, biotin, and the like.

The collagen production promoter according to the present invention can be used as an additive for cosmetics, external pharmaceuticals, or external quasi-drugs. The cosmetic composition or external preparation for skin according to the present invention is used in a form to be applied to the skin (for example, by coating or spraying). Specifically, the cosmetic composition or external preparation for skin according to the present invention is used as a cosmetic, an external pharmaceutucal, or an external quasi-drug. Among these, the cosmetic is preferred in terms of promoting collagen production on a daily basis. Such a cosmetic or external preparation includes various hair cosmetics such as hair tonics or hair growth agents, or shampoos, rinses, and hair lotions (including tonics and liquids) that are effective for hair tonic or hair growth.

The form of the cosmetic composition or the external preparation for skin according to the present invention is not particularly limited as long as it can be applied to the skin or mucous membranes, and examples include pastes, mousses, gels, liquids, emulsions, suspensions, creams, ointments, sheets, aerosols, sprays, and liniments. In particular, when it is used as a cosmetic, examples include lotions; emulsions such as emollient emulsions, milky lotions, nourishing emulsions, and cleansing emulsions; creams such as emollient creams, massage creams, cleansing creams, and makeup creams. Further, in particular, when it is used as a hair care product such as hair growth agents and hair restoration agents, examples include tonics, hair creams, hair lotions, aerosols (sprays), mousses, shampoos, conditioners, and liquids.

The cosmetic composition or the external preparation for skin according to the present invention can be used by directly applying, spraying, or pasting it onto the skin or mucous membranes as a cosmetic, an external pharmaceutical, or an external quasi-drug. The rate of use can be appropriately selected depending on the user (age, sex, purpose, severity of symptoms in the affected area, and the like, for human) and is not particularly limited. For example, an effective amount to promote collagen production may be transdermally administered to the skin from once per day to five times per day.

An amount of application when applying the cosmetic composition or the external preparation for skin according to the present invention is preferably 1 mg or more per dose as converted to the pyrimidine nucleotide or its precursor, more preferably 1 to 1000 mg, and even more preferably 5 to 800 mg, and still more preferably 10 to 500 mg.

The preparation for oral administration according to the present invention can be subjected to practical use as a medicine, a supplement, an enteral nutrient, and so on. In this case, the pyrimidine nucleotide or the precursor thereof can be formulated alone or in combination with a formulation auxiliary or the like

The preparation may be in the form of a tablet, granule, capsule, granule, powder, solution, syrup, emulsion, and the like.

In the preparation described above, in addition to the active ingredient according to the present invention, any formulation auxiliaries such as excipients, binders, disintegrants, lubricants, flavoring agents, solubilizing aids, suspending agents, coating agents, and the like can be appropriately combined according to each delivery form.

Although an amount of the preparation for oral administration according to the present invention to be administered or ingested varies depending on the age, body weight, and severity of symptoms of the subject, the method of administration or ingestion, and the like, the amount administered per dosage may preferably be 1 to 5000 mg, and more preferably 1 to 1000 mg.

As shown in Examples below, the present invention promotes collagen production in fibroblasts through epidermal cells. This is completely different from the previously known attributes and suitable uses of the pyrimidine nucleotides. For example, Patent Literature 1 (WO 2005/034902) teaches that "UMP 2Na itself has no effect of promoting collagen production". Naturally, pyrimidine nucleotides that have no effect of promoting collagen production have been thought to be unsuitable for collagen production promoting applications.

The reason why the pyrimidine nucleotides have the effect of promoting collagen production, which is completely opposite to the previous findings, would be caused by the finding of a new mechanism of action using an experimental system different from previous experimental systems. That is, in the previous findings such as Patent Literature 1, the fibroblasts are directly treated with UMP, but in this case, the effect of promoting collagen production is not exerted at all. On the other hand, it is found from Examples of this application that a significant effect of promoting collagen production can be exerted by treating the epidermal cells with the pyrimidine nucleotide, and treating its culture supernatant with the fibroblasts. When it is used as an external preparation for skin, it is naturally used for the epidermis, so that the findings of the present invention can be the discovery of the true value for the pyrimidine nucleotide as an external preparation for skin.

As is clear from the contents previously described, the present invention is based on the findings that the pyrimidine nucleotide or the precursor thereof has a novel property of promoting collagen production in fibroblasts through epidermal cells, and that the property allows the pyridine nucleotide to be suitable for a novel application of a collagen production promoter. Therefore, the present invention is illustrated below:
[1] A collagen production promoter comprising at least one pyrimidine nucleotide or precursor thereof as an active ingredient.
[2] The collagen production promoter according to [1], wherein the pyrimidine nucleotide or precursor thereof is not at least one purine-based nucleic acid-related substance selected from the group consisting of adenine, adenosine, adenosine phosphate esters, hypoxanthine, inosine, inosinic acid, and salts thereof.
[3] The collagen production promoter according to [1] or [2], wherein a concentration of the pyrimidine nucleotide or precursor thereof is 0.025 to 10.0 (w/v%).
[4] A cosmetic composition or external preparation for skin for promoting collagen production, the cosmetic composition or external preparation for skin comprising at least one pyrimidine nucleotide or precursor thereof as an active ingredient.
[5] The cosmetic composition or external preparation for skin according to [4], wherein a concentration of the pyrimidine nucleotide or precursor thereof is 0.025 to 10.0 (w/v%).
[6] A preparation for oral administration for promoting collagen production, the preparation comprising at least one pyrimidine nucleotide or precursor thereof as an active ingredient.
[7] The preparation for oral administration according to [6], wherein a concentration of the pyrimidine nucleotide or precursor thereof is 0.025 to 10.0 (w/v%).
[8] A method for promoting collagen production in a skin, the method comprising applying the cosmetic composition or external preparation for skin according to [4] or [5] to the skin.
[9] The method for promoting collagen production in a skin according to [8], wherein an amount of the cosmetic composition or external preparation for skin applied is 1 to 1000 mg per dosage, as converted to an amount of the pyrimidine nucleotide or precursor thereof.
[10] A method for promoting collagen production, the method comprising orally administering the preparation for oral administration according to [6] or [7].
[11] The method according to [10], wherein an administered amount of the preparation for oral administration is 1 to 5000 mg per dosage.
[12] A pyrimidine nucleotide or a precursor thereof, for use in promoting collagen production.
[13] Use of at least one pyrimidine nucleotides or precursor thereof, for promoting collagen production.

### [Examples]

While the present invention will be described below with reference to Examples, the present invention is not limited to these Examples in any way.

### (Example 1) Evaluation of Effect of Promoting Collagen Production on Fibroblasts Using Culture Supernatant from Three-dimensional Cultured Epidermis

To reproduce the phenomenon that would occur in actual epidermis, the effect of promoting collagen production for pyrimidine nucleotides was evaluated with a culture supernatant from three-dimensional cultured epidermis, and with fibroblasts.

The experiment was conducted according to the following procedure.

### (Acquisition of Culture Supernatant fro Three-dimensional Cultured Epidermis)

Three-dimensional cultured epidermis (LabCyte EPI MODEL 24; manufactured by J TEC) was set in a 24-well plate to which 500 µL of the attached assay medium was added, and conditioned overnight at 37°C. 50 µL of sample-containing PBS (-) was applied from the stratum corneum side and cultured for 24 hours. After removing the sample-containing PBS (-), the plate was washed with PBS (-). The samples to be treated with UVB were irradiated with 600 mJ/cm² using a UVB lamp (TL20W/12RS; manufactured by PHILIPS). For both samples with and without the UVB treatment, the media were replaced with fresh media, 50 µL of sample-containing PBS (-) was newly applied from the stratum corneum side, and further cultured for 48 hours. After culturing, the culture supernatant was collected. The concentrations of CMP, 2Na in the respective samples were 0, 0.125, 0.25, 1.0 and 2.0 (w/v%), respectively.

### (Treatment of Culture Supernatant with Normal Human Fibroblasts)

Normal human fibroblasts (NHDF; Kurabo) were seeded in a 96-well plate at a density of 2.5 x 10⁴ cells/well/100 µL and cultured at 37°C for 24 hours. The cells were then replaced with 100 µL of the culture supernatant obtained in the culture supernatant acquisition step for the three-dimensional cultured epidermis as described above and cultured for 24 hours.

### (Quantification of Type I Collagen)

The culture supernatant from normal human fibroblasts was collected and an amount of type I collagen was quantified by ELISA. The protein amount of the culture supernatant was also measured by BCA protein assay.

The quantified results of the amount of type I collagen are shown in FIG. 1.

Regardless of whether or not the samples were irradiated with UVB, the amount of collagen in the culture supernatant from normal human fibroblasts was increased by the treatment with the culture supernatant from the three-dimensional cultured epidermis, depending on the concentration of CMP, 2Na used to treat the three-dimensional cultured epidermis (FIG. 1).

The results of this Example demonstrated that the collagen production by fibroblasts was promoted by treating the three-dimensional cultured epidermis with CMP, 2Na from the stratum corneum side, and then treating the fibroblasts with the culture supernatant.

### (Example 2) Evaluation of mRNA Expression Levels of Collagen Synthesis-related Factors in Fibroblasts Using Culture Supernatant from Three-dimensional Cultured Epidermis

Similarly, the mRNA expression levels of Collagen 1A1 (COL1A1), a factor related to type I collagen synthesis, were quantified by real-time PCR.

### (Acquisition of Culture Supernatant from Three-dimensional Cultured Epidermis)

Three-dimensional cultured epidermis (LabCyte EPI MODEL 24; manufactured by J TEC) was set in a 24-well plate to which 500 µL of the attached assay medium was added, and conditioned overnight at 37°C. 50 µL of sample-containing PBS (-) was applied from the stratum corneum side and cultured for 24 hours. After removing the sample-containing PBS (-), the plate was washed with PBS (-). The medium was replaced with a fresh medium, 50 µL of sample-containing PBS (-) was newly applied from the stratum corneum side, and further cultured for 48 hours. After culturing, the culture supernatant was collected. The concentrations of CMP, 2Na in the respective samples were 0, 1.0, and 2.0 (w/v%), respectively.

### (Treatment of Normal Human Fibroblasts with Culture Supernatant and Quantification of mRNA Expression Level of Collagen 1A1)

Normal human fibroblasts were treated with the culture supernatant by the same method as that of Example 1. RNA was extracted from NHDF using RNeasy Mini Kit (QIAGEN), and mRNA expression analysis for collagen 1A1 (COL1A1) was performed by real time PCR (ΔΔCt method). Glyceraldehyde 3-phosphate dehydrogenase (GAPDH) was used as an endogenous control.

The results of the analysis of COL1A1 mRNA expression are shown in FIG. 2.

It was revealed that the expression level of COL1A1 was increased by treating the three-dimensional cultured epidermis with the culture supernatant, depending on the concentration of CMP, 2Na used to treat the three-dimensional cultured epidermis.

The results of this Example demonstrated that the expression level of the collagen synthesis-related factor in fibroblasts was increased by treating the three-dimensional cultured epidermis with CMP, 2Na from the stratum corneum side, and then treating the culture supernatant with the fibroblasts.

### (Example 3) Evaluation of Effect of Promoting Collagen Production in Fibroblasts Using Normal Human Epidermal Cell Culture Supernatant

The effects of promoting collagen production for pyrimidine nucleotides were then evaluated with the culture supernatant from normal human epidermal cells and with fibroblasts. In this evaluation, the effects were compared for the case where the fibroblasts were directly treated with pyrimidine nucleotides and the case where the fibroblasts were treated with the normal human epidermal cell culture supernatant treated with the pyrimidine nucleotides.

### (Acquisition of Culture Supernatant of Normal Human Epidermal Cells)

Normal human epidermal cells (NHEK; Kurabo) were seeded in a 96-well plate at a density of 2.5 x 10⁴ cells/well and cultured at 37°C for 24 hours. The medium was replaced with 100 µL of sample-containing medium (HuMedia-KG2; Kurabo) and cultured for 24 hours. After culturing, the culture supernatant was collected. The sample solutions used were 0, 1.25, 2.5, and 5 (mM) (i.e., 0, 0.0459, 0.0918, and 0.184 (w/v%)) of CMP, 2Na, and 0, 1.25, 2.5, and 5 (mM) (i.e., 0, 0.046, 0.092, and 0.184 (w/v%)) of UMP, 2Na.

### (Treatment of Normal Human Fibroblasts with Culture Supernatant)

Normal human fibroblasts (NHDF; Kurabo) were seeded in a 96-well plate at a density of 2.5 x 10⁴ cells/well/100 µL and cultured at 37°C for 24 hours. The medium was then replaced with 100 µL of the culture supernatant obtained in the culture supernatant acquisition step from the three-dimensional cultured epidermis as described above and cultured for 24 hours.

### (Direct Treatment of Normal Human Fibroblasts with Sample)

Normal human fibroblasts (NHDF; Kurabo) were seeded in a 96-well plate at a density of 2.55 x 10⁴ cells/well/100 µL and cultured at 37°C for 24 hours. The medium was then replaced with 100 µL of media (HuMedia-KG2; Kurabo) containing CMP, 2Na at 0, 1.25, 2.5, 5 (mM) (i.e., 0, 0.0459, 0.0918, 0.184 (w/v%)) and UMP, 2Na at 0, 1.25, 2.5, 5 (mM) (i.e., 0, 0.046, 0.092, 0.184 (w/v%)), and cultured for 24 hours.

### (Quantification of Type I Collagen)

The culture supernatant of normal human fibroblasts was collected and an amount of type I collagen was quantified by ELISA. The protein amount of the culture supernatant was also measured by BCA protein assay.

The quantified results of the amount of type I collagen are shown in FIG. 3 and FIG. 4.

The amount of collagen in the culture supernatant of normal human fibroblasts was increased depending on the concentration of CMP, 2Na added to the normal human epidermal cell culture medium (FIG. 3). On the other hand, no increase in the amount of collagen was observed in the group where the normal human fibroblasts were directly treated with CMP, 2Na.

The amount of collagen in the culture supernatant from normal human fibroblasts was increased depending on the concentration of UMP, 2Na added to the normal human epidermal cell culture medium (FIG. 4). On the other hand, no increase in the amount of collagen was observed in the group where the normal human fibroblasts were directly treated with UMP, 2Na.

The results of this Example strongly suggest that when the external preparation for skin, containing the pyrimidine nucleotide, is applied to the skin, it acts on fibroblasts through epidermal cells, thereby promoting collagen production in the fibroblasts.

When comparing the effects of promoting collagen production for cytidylic acid and uridylic acid, cytidylic acid had a higher effect of promoting collagen production.

On the other hand, it was confirmed that when the fibroblasts were directly treated with the pyrimidine nucleotides, no effect of promoting collagen production was observed, as previously reported in WO 2005/034902 A1 and the like.

### (Example 4) Ex Vivo Evaluation Test Using Freshly Extracted Human Skin

To evaluate the effect on actual skin with greater precision, an *ex vivo* evaluation test was conducted using freshly extracted human skin.

To evaluate the effect on actual human skin with greater precision, an *ex vivo* evaluation test was conducted using freshly extracted human skin.

Abdominal skin tissue was used, which was taken from a 39-year-old Caucasian female patient during cosmetic surgery, after obtaining informed consent for use in research. The extracted skin was placed in a culture plate supplemented with 1.3 ml of D-MEM/Ham's F-12 medium, and conditioned overnight at 37°C in 5% CO₂. An appropriate amount of PBS(-) containing a given concentration of each sample was applied from the stratum corneum side, and further cultured for 24 hours. The treatment was continued for a total of 72 hours while replacing the sample and medium with fresh ones every 24 hours. The concentrations of CMP, 2Na in the respective samples were 0, 1.0, and 2.0 (w/v%) .

After the cultured skin tissue was fixed according to a standard method, skin tissue sections were prepared, collagen fibers were stained with elastica-van Gieson stain, and observed under a microscope.

The results of tissue analysis by elastica-van Gieson staining are shown in FIG. 5.

In the fresh human extracted skin, the cytidylic acid treatment also tended to result in denser collagen fibers.

The results of this Example strongly suggest that collagen production is also promoted by pyrimidine nucleotides or precursors thereof in actual skin.

## Claims

1. A collagen production promoter comprising at least one pyrimidine nucleotide or precursor thereof as an active ingredient.

2. The collagen production promoter according to claim 1, wherein the pyrimidine nucleotide or precursor thereof is not at least one purine-based nucleic acid-related substance selected from the group consisting of adenine, adenosine, adenosine phosphate esters, hypoxanthine, inosine, inosinic acid, and salts thereof.

3. The collagen production promoter according to claim 1 or 2, wherein a concentration of the pyrimidine nucleotide or precursor thereof is 0.025 to 10.0 (w/v%).

4. A cosmetic composition or external preparation for skin for promoting collagen production, the cosmetic composition or external preparation for skin comprising at least one pyrimidine nucleotide or precursor thereof as an active ingredient.

5. The cosmetic composition or external preparation for skin according to claim 4, wherein a concentration of the pyrimidine nucleotide or precursor thereof is 0.025 to 10.0 (w/v%).

6. A preparation for oral administration for promoting collagen production, the preparation comprising at least one pyrimidine nucleotide or precursor thereof as an active ingredient.

7. The preparation for oral administration according to claim 6, wherein a concentration of the pyrimidine nucleotide or precursor thereof is 0.025 to 10.0 (w/v%).

8. A method for promoting collagen production in a skin, the method comprising applying the cosmetic composition or external preparation for skin according to claim 4 or 5 to the skin.

9. The method for promoting collagen production in a skin according to claim 8, wherein an amount of the cosmetic composition or external preparation for skin applied is 1 to 1000 mg per dosage, as converted to an amount of the pyrimidine nucleotide or precursor thereof.

10. A method for promoting collagen production, the method comprising orally administering the preparation for oral administration according to claim 6 or 7.

11. The method according to claim 10, wherein an administered amount of the preparation for oral administration is 1 to 5000 mg per dosage.

12. A pyrimidine nucleotide or a precursor thereof, for use in promoting collagen production.

13. Use of at least one pyrimidine nucleotides or precursor thereof, for promoting collagen production.
